Europäisches Patentamt

European Patent Office

Office européen des brevets

(11) Publication number: **0 148 540**
**B1**

# EUROPEAN PATENT SPECIFICATION

(45) Date of publication of patent specification: **19.04.89**

(21) Application number: **84300131.4**

(22) Date of filing: **10.01.84**

(51) Int. Cl.⁴: **B 01 J 29/28,** C 07 C 5/27,
C 07 C 15/08

(54) Active zeolite catalysts of improved stability.

(43) Date of publication of application:
**17.07.85 Bulletin 85/29**

(45) Publication of the grant of the patent:
**19.04.89 Bulletin 89/16**

(84) Designated Contracting States:
**BE DE FR GB IT NL SE**

(56) References cited:
**EP-A-0 021 604**
**EP-A-0 034 444**
**EP-A-0 036 704**
**FR-A-2 310 963**
**US-A-3 965 209**
**US-A-4 356 338**

(73) Proprietor: **MOBIL OIL CORPORATION**
**150 East 42nd Street**
**New York New York 10017 (US)**

(72) Inventor: **Chester, Arthur Warren**
**517 Country Club Drive**
**Cherry Hill New Jersey 08003 (US)**
Inventor: **Chu, Yung-Feng**
**121 Randle Drive**
**Cherry Hill New Jersey 08034 (US)**

(74) Representative: **Cooper, John Anthony et al**
**Mobil Court 3 Clements Inn**
**London WC2A 2EB (GB)**

The file contains technical information
submitted after the application was filed and
not included in this specification

Courier Press, Leamington Spa, England.

EP 0 148 540 B1

**Description**

This invention relates to a process for resteaming a zeolite catalyst so as to substantially retain its initial activity and to a process for the preparation of superior zeolite catalysts to be used in acid-catalyzed reactions.

Acid-catalyzed reactions, e.g., xylene isomerization, toluene disproportionation, etc., result in the rapid degeneration of catalyst activity. It is well known to the art that mild-to-severely steamed zeolite catalysts provide improved stability (see U.S. Patent No. 4,356,338) but suffer from lowered activity in acid-catalyzed reactions.

Much of the prior art in this area deals with severely steamed zeolite catalysts in reactions such as xylene isomerization.

U.S. Patent No. 4,224,141 discloses a xylene isomerization process with a catalyst steamed at a temperature in excess of 538°C (1000°F) for a period of time longer than 15 hours. The resulting catalyst is highly stable, but suffers from lowered activity.

U.S. Patent No. 4,188,282 discloses a xylene isomerization process using a catalyst with a silica/alumina ratio of at least 200. The catalyst is severely steamed to a lowered activity and described in U.S. Patent No. 4,016,218 and U.S. Patent No. 3,965,209.

U.S. Patent No. 4,236,996 discloses a xylene isomerization process wherein the catalyst is steamed at a high temperature to reduce the activity such that the conversion reaction temperature must be increased by at least 28°C (50°F) to equal the conversion capability of an unsteamed zeolite.

U.S. Patent No. 3,965,209 discloses a process whereby the zeolite is steamed to reduce the alpha activity to less than 500 by treating the zeolite in a steam atmosphere at a temperature of from 250°C to about 1000°C (526°F to about 2026°F) for from about 1/2 hours to 100 hours.

According to the present invention a method of increasing the stability of an unsteamed catalyst comprising a zeolite having a silica/alumina mole ratio greater than 12 and a constraint index of 1 to 12 comprises steaming said catalyst under controlled conditions of temperature, time and steam partial pressure so as to increase the initial $\alpha$-activity ($\alpha_i$) of said catalyst and to produce a steamed catalyst having a peak $\alpha$-activity ($\alpha_p$), and is characterised in that the steaming is continued to subsequently reduce the $\alpha$-activity from said peak $\alpha$-activity to an $\alpha$-activity ($\alpha$) which is defined by:

$$1 > \frac{\alpha}{\alpha_i} > 0.75$$

Such a steamed catalyst has enhanced cycle length relative to the unsteamed catalyst.

The present invention makes possible an improved process for the catalytic conversion of an organic reactant involving an acid-catalyzed reaction using a catalyst comprising a zeolite having a silica/alumina ratio greater than 12 and a constraint index of 1 to 12. Such a process comprises contacting the reactant with such a zeolite-containing catalyst which has been subjected in its unsteamed state to a steaming procedure under controlled conditions of temperature, time and steam partial pressure so as to initially increase the $\alpha$-activity of said catalyst and produce a steamed catalyst having a peak $\alpha$-activity, and to subsequently reduce the $\alpha$-activity from said peak $\alpha$-activity below, but no more than 25% below, the initial $\alpha$-activity of said unsteamed catalyst. The catalyst, when steamed in this manner, has enhanced cycle length relative to the unsteamed catalyst.

The organic reactant conversion process advantageously involves isomerizing the xylene content of a charge mixture of eight carbon atom aromatic hydrocarbon compounds, which mixture contains xylenes and ethylebenzene, by contacting the mixture under conversion conditions with a catalyst comprising a zeolite having a silica/alumina ratio greater than 12 and a constraint index of 1 to 12. Such a xylene isomerization process utilizes such a catalyst which has been steamed in a particular manner while maintaining conversion temperature of about 260°C to 538°C. Prior to contact with the charge mixture, the zeolite-based catalyst is steamed at a temperature and pressure and for a period of time so as to initially increase the $\alpha$-activity of the catalyst and produce a steamed catalyst having a peak $\alpha$-activity and to subsequently reduce the $\alpha$-activity from said peak $\alpha$-activity no less than 75% of the initial $\alpha$-activity of the fresh, unsteamed zeolite-based catalyst. The catalyst, when steamed in this manner, has enhanced stability over the fresh catalyst.

This invention is accomplished by presteaming a fresh zeolite catalyst under mild conditions until the activity of the mildly steamed catalyst has been increased to a peak and then reduced to a level which is substantially equivalent to that of a fresh, unsteamed catalyst. The siliceous crystalline zeolites used in such catalysts are members of a class of zeolites that exhibits unusual properties. Such zeolite materials are those which have a silica to alumina molar ratio of at least 12 and a constraint index within the range of 1 to 12. Zeolite materials of this type are well known. Such zeolites and their use as catalysts for conversion of aromatic hydrocarbons are generally described, for example, in the aforementioned U.S. Patent No. 4,236,996. Crystalline zeolites of the type useful in the catalysts of the present invention include, ZSM-5, ZSM-11, ZSM-12, ZSM-23, ZSM-35, ZSM-38 and ZSM-48, with ZSM-5 being particularly preferred.

2

ZSM-5 is described in greater detail in U.S. Patent Nos. 3,702,886 and Re 29,948, which patents provide the X-ray diffraction pattern of the therein disclosed ZSM-5.

ZSM-11 is described in U.S. Patent No. 3,709,979, which discloses in particular the X-ray diffraction pattern of ZSM-11.

ZSM-12 is described in U.S. Patent No. 3,832,449, which discloses in particular the X-ray diffraction pattern of ZSM-12.

ZSM-23 is described in U.S. Patent No. 4,076,842, which discloses in particular the X-ray diffraction pattern for ZSM-23.

ZSM-35 is described in U.S. Patent No. 4,016,245, which discloses in particular the X-ray diffraction pattern for ZSM-35.

ZSM-38 is described in U.S. Patent No. 4,046,859, which discloses in particular the X-ray diffraction pattern for ZSM-38.

ZSM-48 is more particularly described in European Patent Publication EP—A—0015132 which includes the X-ray diffraction pattern for ZSM-48.

When synthesized in the alkali metal form, the zeolite used to form the catalysts herein can be conveniently converted in a conventional manner to the hydrogen form, generally by intermediate formation of the ammonium form as a result of ammonium ion exchange and calcination of the ammonium form to yield the hydrogen form of the zeolite. In addition to the hydrogen form, other forms of the zeolite can be employed in the catalyst compositions herein so long as the original alkali metal has been reduced to less than about 50% by weight of the original alkali metal contained in the zeolite as-synthesized, usually 0.5% by weight or less. Thus, the original alkali metal of the zeolite may be replaced by ion exchange with other suitable metal cations of Groups I through VIII of the Periodic Table, including, by way of example, nickel, copper, zinc, palladium, calcium or rare earth metals.

In preparing the zeolite-containing catalysts used in the present invention, the above-described siliceous crystalline zeolite material can be combined with an inorganic oxide binder or matrix comprising another material resistant to the temperature and other conditions employed in the organic reactant conversion process embodiments of the present invention. Such matrix material is useful as a binder and imparts greater resistance to the catalyst for the severe temperature, pressure and reactant feed stream velocity conditions encountered in such processes.

Useful matrix materials include both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the binder for the siliceous crystalline zeolite material employed herein can comprise a porous matrix material, such as alumina, silica-alumina, silica-magnesia, silica-zirconia, silica-thoria, silica-beryllia, and silica-titania, as well as ternary compositions, such as silica-alumina-thoria, silica-alumina zirconia, silica-alumina-magnesia and silica-magnesia-zirconia. The matrix may be in the form of a cogel. The relative proportions of zeolite component and inorganic oxide gel matrix, on an anhydrous basis, may vary widely with the zeolite content ranging from between about 1 to about 99 percent by weight and more usually in the range of about 25 to about 80 percent by weight of the dry catalyst.

The zeolite-based catalysts hereinbefore described are known to be useful in promoting a wide variety of organic compound conversion reactions including, for example, processing of aromatic hydrocarbons. U.S. Patent No. 4,163,028, for instance, describes a method of processing $C_8$ aromatics for isomerization of xylene and conversion of ethylbenzene. According to that patent, the reactions are conducted at temperatures of 427°C to 537°C (800°F to 1000°F) with a zeolite having a constraint index of 1 to 12 and a very high silica/alumina ratio which may be as high as or higher than 3000. Such catalyst have low acid activity by reason of the small number of sites capable of being rendered protonic by ammonium exchange and calcination.

The improvement over the prior art which forms the basis of the present invention is predicated upon the discovery that it is not necessary to severely reduce the activity of zeolite catalyst by steaming in order to obtain enhanced stability. It has been found that, by mildly presteaming a fresh zeolite catalyst under controlled conditions, the catalyst will initially exhibit an increase in activity followed by a gradual decline. When the activity of the catalyst becomes substantially similar to that of the fresh, unsteamed catalyst, the steam treatment is terminated. The resulting catalyst has an activity level substantially similar to that of fresh, unsteamed catalyst together with improved stability.

Examples 1—10

Table 1 illustrates the effect of very mild presteaming on fresh zeolite catalyst for use in xylene isomerization. The catalyst employed in each example is HZSM-5 with a silica/alumina ratio of 70. In Table 1, α represents the degree of activity of the mildly steamed catalyst; α₀ represents the degree of activity of

3

# EP 0 148 540 B1

the fresh, unsteamed catalyst, and $\alpha/\alpha_o$ represents the degree in which $\alpha$ increases over or decreases below $\alpha_o$.

As is well known in the art, the $\alpha$-activity gives an approximate indication of the catalytic cracking activity of the catalyst compared to a standard catalyst and it gives the relative rate constant (rate of normal hexane conversion per volume of catalyst composition per unit time). It is based on the activity of the highly active silica alumina cracking catalyst taken as an $\alpha$ of 1. This test is described in U.S. Patent No. 3,354,078 and in *The Journal of Catalysis*, Vol. 4, pp. 522—529, August 1965. For purposes of the present invention, however, all measurements of $\alpha$ are to be made at 538°C (1000°F) and all references to $\alpha$ are to be understood to refer to the value obtained when the hexane cracking is measured at 538°C (1000°F).

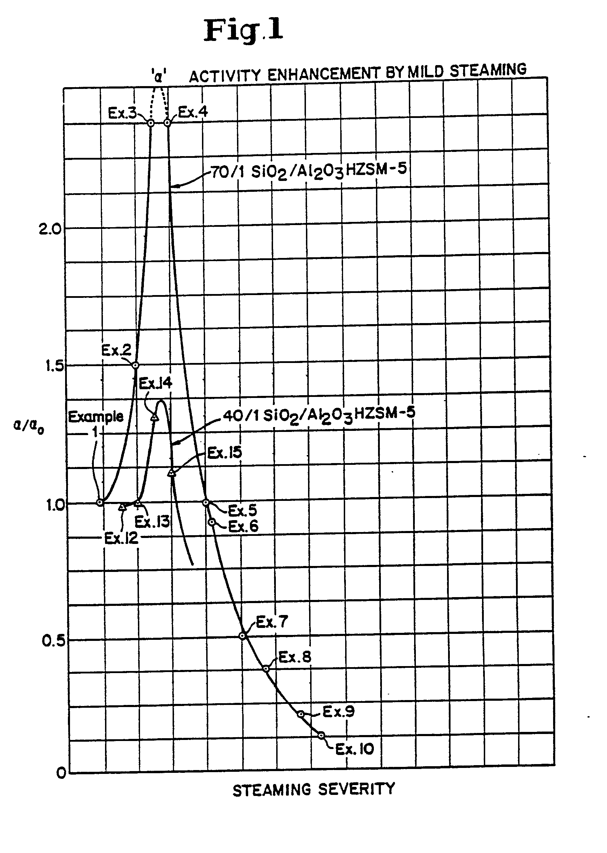

Illustrated in Figure 1 is the relationship of activity of the mildly presteamed catalyst to the steaming severity of the catalyst. The parameters for steaming severity are represented in Table 1. It is understood that the term "steaming severity" represents a proportional relationship between the length of time, the temperature, the partial pressure and the percent of steam in the steam treatment. As is shown in Figure 1, an increase in steaming severity resulted in an increase in $\alpha$-activity of the catalyst to a point of peak enhancement (represented by Example 3, Table 1). With continued increases in steaming severity, the $\alpha$-activity decreased. At the level of severity represented by Example 5, Table 1, the $\alpha$-activity of the catalyst was substantially equivalent to that of the fresh catalyst ($\alpha=.9\alpha_o$). As can be readily seen from Examples 7—10, increased steaming severity further diminished the $\alpha$-activity of the zeolite catalyst.

## TABLE 1

### Effect of steaming of HZSM-5

| Example No. | 1 | 2 | 3 | 4 | 5 | 6 | 7 | 8 | 9 | 10 |
|---|---|---|---|---|---|---|---|---|---|---|
| Steam treat | None | 100% | 100% | 100% | 100% | 100% | 100% | 100% | 100% | 100% |
| Hours | Fresh | 6 | 6 | 6 | 3 | 6 | 6 | 8 | 3.5 | 8 |
| Temp. °C | Fresh | 204 | 260 | 316 | 427 | 427 | 454 | 468 | 538 | 552 |
| Press. kPa | Fresh | 101 | 101 | 101 | 101 | 101 | 101 | 101 | 101 | 101 |
| $\alpha$ | 160* | 240 | 380 | 360 | 148 | 130 | 82 | 60 | 33 | 20 |
| $\alpha/\alpha_o$ | 1 | 1.5 | 2.4 | 2.3 | 0.9 | 0.8 | 0.5 | 0.4 | 0.2 | 0.1 |

\*$\alpha=\alpha_o$

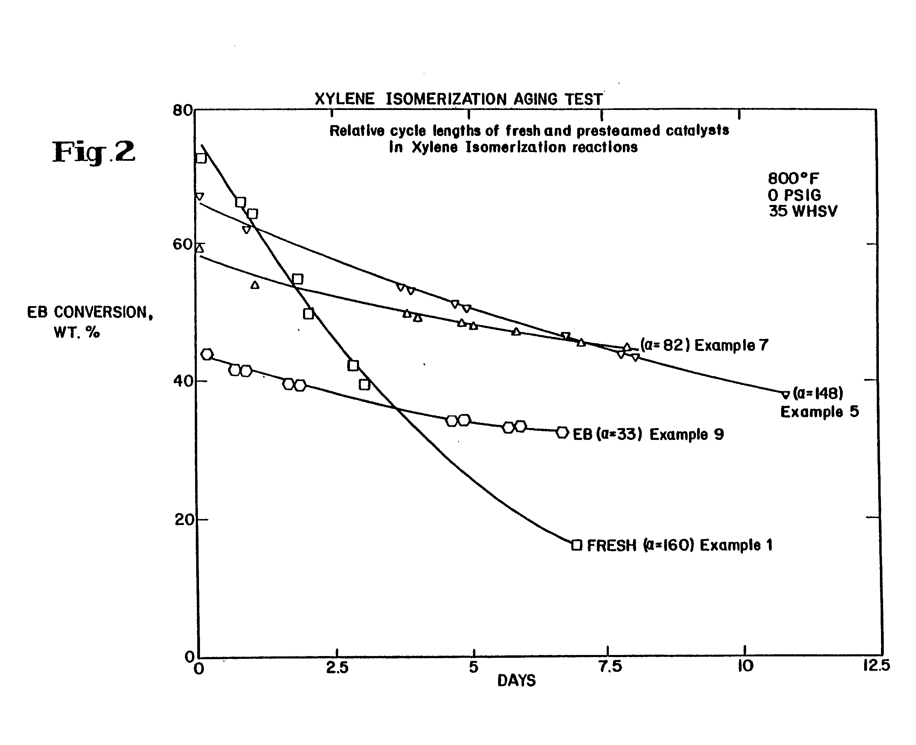

The import of mildly presteaming a zeolite catalyst is disclosed in Figure 2 which illustrates the relative cycle lengths of fresh and presteamed catalysts in xylene isomerization reactions. Under similar isomerization conditions, the presteamed catalysts ($\alpha=148$) continued to operate at least 3 times longer per run. As the steaming severity increased (Examples 6—10), the resultant catalysts demonstrated increased stability. However, as is shown in Table 1, the catalyst activity diminished. Thus, mildly steaming a fresh zeolite catalyst under conditions such that the steaming conditions result in a catalyst having an $\alpha$-activity no less than 75% and, preferably, greater than 85% of the activity of a fresh unsteamed catalyst and with greatly enhanced stability is desired for the present invention.

Examples 11—15

Table 2 and Figure 1 show the effect of mildly presteaming a zeolite catalyst, e.g., HZSM-5, silica/alumina ratio 40. Such a catalyst is desirable for the conversion of propane to aromatics.

4

TABLE 2

| Effect of presteaming HZSM-5 | | | | | |
|---|---|---|---|---|---|
| Example No. | 11 | 12 | 13 | 14 | 15 |
| Steam treat | None | 100% | 100% | 100% | 100% |
| Hours | Fresh | 6 | 6 | 6 | 6 |
| Temp. °C | Fresh | 149 | 204 | 260 | 316 |
| Press. kPa | Fresh | 101 | 101 | 101 | 101 |
| $\alpha$ | 550* | 520 | 540 | 740 | 600 |
| $\alpha/\alpha_o$ | 1 | 0.95 | 1.0 | 1.3 | 1.1 |

*$\alpha_o$

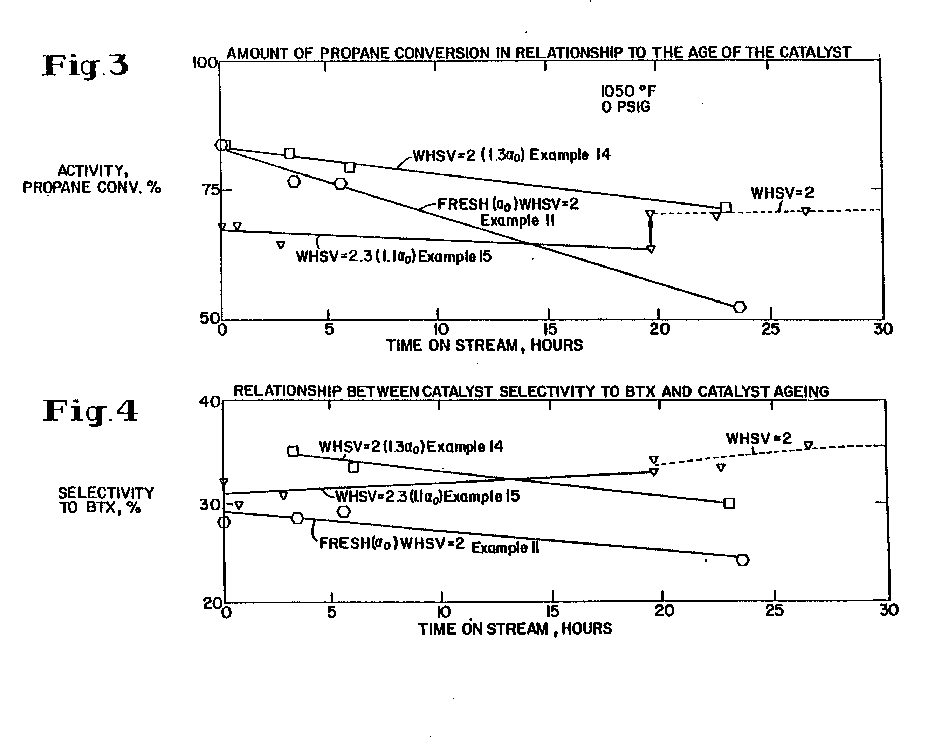

Figure 3 represents the relationship of the amount of propane conversion to the age of the catalyst (time on stream) for three catalysts illustrated by Examples 11, 14 and 15 of Table 2. The broken curves represent the conversion level (Figure 3) and selectivity level (Figure 4) of the optimally steamed catalyst (Example 15) after its WHSV was changed from 2.3 to 2 and the catalyst remained on stream for an additional 20 hours. Although the initial activity of the optimally steamed catalyst was slightly less than that for either the fresh catalyst (Example 11) or the mildly steam treated catalyst (Example 14), the activity of the optimally steamed catalyst showed greater stability than either of the other catalysts.

Figure 4 represents the relationship between catalyst selectivity to BTX (Benzene, Toluene, Xylene) and catalyst aging for three catalysts. The selectivity to BTX of the catalyst of Example 15 was greater than that of the fresh catalyst (Example 11). The initial selectivity of the catalyst of Example 14 was slightly greater than the optimally steamed catalyst of Example 15, however, the optimally steamed catalyst shows a greater stability.

The results of these experiments show that mild presteaming of a catalyst to place its $\alpha$-activity past peak enhancement but no less than 75% and, preferably, within 10% of the initial $\alpha$-activity is essential for a highly stable catalyst of high activity and selectivity.

The zeolite catalysts to be steamed according to the invention are generally those zeolites of at least 12 silica/alumina ratio and a constraint index of 1 to 12 which, in the acid form, have activity to convert about 30% of the ethylbenzene in a mixture thereof with xylenes under the isomerization conditions of the aforementioned U.S. Patent No. 3,856,872, say 316°C—371°C (600°—700°F). The degree of steaming should be such that the $\alpha$-activity of the steamed catalyst should be less than the $\alpha$-activity of the fresh, unsteamed catalyst but no greater than 25% less than the $\alpha$-activity of the fresh catalyst and preferably no greater than 10% less. Under these conditions, the reaction temperature of xylene isomerization reactions should increase by no more than 14°C (25°F) to attain the same ethylbenzene conversion as was observed before steaming. The reaction of the present invention will be conducted at such elevated temperatures, above 316°C (600°F), as to realize about 30% conversion of the ethylbenzene in the charge. As the temperature is further increased to and above about 343°C (650°F) the reaction of ethylbenzene shifts from disproportionation to dealkylation.

The present invention involves using mild temperature presteaming to partially deactivate the catalyst. The deactivation should be conducted to a level such that the steamed catalyst activity is no less than 75% of the activity of the fresh, unsteamed catalyst and the process requires a maximum 14°C (25°F) rise in operating temperature, well below the minimum 28°C (50°F) rise referred to in U.S. Patent No. 4,236,996. Additionally, the mildly presteamed catalyst demonstrates superior stability characteristics while maintaining catalytic activity substantially equivalent to that of fresh, unsteamed catalysts.

The major importance of this development relates to the use of zeolite catalysts, particularly ZSM-5, in acid catalyzed reactions, e.g., xylene isomerization, propane conversion to BTX, toluene disproportionation, hydrocracking, dewaxing, conversion of alcohols to hydrocarbons such as methanol to gasoline and/or olefins, synthesis gas conversion to fuels, conversion of olefins to heavier fuels, etc. Such reactions would preferably incorporate a catalyst with the unique qualities of stability and catalytic activity similar to fresh, unsteamed catalysts.

For such uses it is possible to combine the zeolites of the present invention with metal promoters such as Zn, ZnPd, Pt, Cr, etc. Such promoters may be incorporated with the zeolite in accordance with the ion exchange technique described hereinbefore or may be incorporated by other techniques such as impregnation. Incorporation of such metal promoters can occur either before or after the zeolite catalysts are steamed in accordance with the present invention.

# EP 0 148 540 B1

**Claims**

1. A method of increasing the stability of an unsteamed catalyst comprising a zeolite having a silica/alumina mole ratio greater than 12 and a constraint index of 1 to 12, said method comprising steaming said catalyst under controlled conditions of temperature, time and steam partial pressure so as to increase the initial $\alpha$-activity $(\alpha_i)$ of said catalyst and to produce a steamed catalyst having a peak $\alpha$-activity $(\alpha_p)$, characterized in that the steaming is continued to subsequently reduce the $\alpha$-activity from said peak $\alpha$-activity and to an $\alpha$-activity $(\alpha)$ which is defined by:

$$1 > \frac{\alpha}{\alpha_i} > 0.75$$

2. A method according to Claim 1, wherein said zeolite is ZSM-5.

3. A method according to Claims 1 or 2 wherein said catalyst further comprises one or more metal promoters.

4. A method according to any of Claims 1 to 3 wherein the silica/alumina ratio of said zeolite is no less than 20.

**Patentansprüche**

1. Verfahren zur Erhöhung der Stabilität eines nicht mit Dampf behandelten Katalysators, der einen Zeolith mit einem Siliciumdioxid/Aluminiumoxid-Molverhältnis von größer als 12 und einem Zwangsindex von 1 bis 12 umfaßt, wobei dieses Verfahren die Dampfbehandlung dieses Katalysators bei geregelten Bedingungen Von Temperatur, Zeit und Dampfpartialdruck umfaßt, um die ursprüngliche $\alpha$-Aktivität $(\alpha_i)$ dieses Katalysators zu erhöhen und einen mit Dampf behandelten Katalysator mit einer $\alpha$-Höchstaktivität $(\alpha_p)$ herzustellen, dadurch gekennzeichnet, daß die Dampfbehandlung fortgeführt wird, um anschließend die $\alpha$-Aktivität von der $\alpha$-Höchstaktivität und bis zu einer $\alpha$-Aktivität $(\alpha)$ zu reduzieren, die durch

$$1 > \frac{\alpha}{\alpha_i} > 0,75 \text{ definiert ist.}$$

2. Verfahren nach Anspruch 1, worin der Zeolith ZSM-5 ist.

3. Verfahen nach den Ansprüche 1 oder 2, worin der Katalysator darüberhinaus einen oder mehrere Metallpromotoren umfaßt.

4. Verfahren nach einem der Ansprüche 1 bis 3, worin das Siliciumdioxid/Aluminiumoxid-Verhältnis des Zeoliths nicht kleiner als 20 ist.

**Revendications**

1. Un procédé pour augmenter la stabilité d'un catalyseur n'ayant pas subit un traitement à la vapeur comprenant une zéolite présentant un rapport molaire silice/alumine supérieur à 12 et un indice de contrainte compris entre 1 et 12, lequel comprend le traitement à la vapeur dudit catalyseur dans les conditions contrôlées de température, de durée et de pression partielle de vapeur, de façon à augmenter l'activité $\alpha$ initiale $(\alpha_i)$ dudit catalyseur et à produire un catalyseur ayant subi un traitement à la vapeur présentant un pic de réactivité $\alpha$ $(\alpha_p)$, caractérisé en ce que le traitement à la vapeur se poursuit pour réduire de façon correspondante l'activité $\alpha$ dudit pic d'activité $\alpha$ jusqu'à une activité $\alpha$ qui est définie par l'équation:

$$1 > \frac{\alpha}{\alpha_i} > 0,75$$

2. Un procédé selon la revendication 1, dans laquelle ladite zéolite est une ZSM-5.

3. Un procédé selon la revendication 1 ou 2, dans laquelle ledit catalyseur comprend en outre un ou plusiers promoteurs métalliques.

4. Un procédé selon l'une quelconque des revendications 1 à 3, dans laquelle le rapport silice/alumine de ladite zéolite n'est pas inférieur à 20.

# Fig.1

ACTIVITY ENHANCEMENT BY MILD STEAMING

Ex.3  Ex.4

70/1 $SiO_2/Al_2O_3$ HZSM-5

Ex.2

Ex.14

Example 1

40/1 $SiO_2/Al_2O_3$ HZSM-5

Ex.15

$a/a_0$

Ex.12

Ex.13

Ex.5
Ex.6

Ex.7

Ex.8

Ex.9

Ex.10

2.0

1.5

1.0

0.5

0

'a'

STEAMING SEVERITY

Fig.2

XYLENE ISOMERIZATION AGING TEST

Relative cycle lengths of fresh and presteamed catalysts
In Xylene Isomerization reactions

800°F
0 PSIG
35 WHSV

EB CONVERSION, WT. %

(α= 82) Example 7

∇ (α=148) Example 5

EB (α=33) Example 9

FRESH (α=160) Example 1

DAYS

EP 0 148 540 B1

Fig.3

ACTIVITY,
PROPANE CONV. %

AMOUNT OF PROPANE CONVERSION IN RELATIONSHIP TO THE AGE OF THE CATALYST

1050 °F
0 PSIG

WHSV=2 (1.3$a_0$) Example 14

FRESH ($a_0$) WHSV=2
Example 11

WHSV=2

WHSV=2.3 (1.1$a_0$) Example 15

TIME ON STREAM, HOURS

Fig.4

SELECTIVITY
TO BTX, %

RELATIONSHIP BETWEEN CATALYST SELECTIVITY TO BTX AND CATALYST AGEING

WHSV=2 (1.3$a_0$) Example 14

WHSV=2

WHSV=2.3 (1.1$a_0$) Example 15

FRESH($a_0$) WHSV=2 Example 11

TIME ON STREAM, HOURS

EP 0 148 540 B1